# EUROPEAN PATENT SPECIFICATION

(11) **EP 0 758 233 B1**
(45) Date of publication and mention of the grant of the patent: **03.12.2003**
(21) Application number: 95915983.1
(22) Date of filing: 21.04.1995
(51) Int. Cl.: A61K 31/40, A61K 38/21, A61P 25/00

(54) **PDE IV INHIBITORS FOR TREATING MULTIPLE SCLEROSIS**
PDE IV-INHIBITOREN ZUR BEHANDLUNG DER MULTIPLEN SKLEROSE
INHIBITEURS DE PDE IV UTILISES DANS LE TRAITEMENT DE LA SCLEROSE EN PLAQUES

(30) Priority: 21.04.1994 US 231969; 02.06.1994 US 253938; 21.10.1994 US 327478; 15.11.1994 US 340416
(43) Date of publication of application: 19.02.1997
(60) Divisional application: 03090246.4
(73) Proprietor: SCHERING AKTIENGESELLSCHAFT, 13342 Berlin (DE)
(72) Inventor: PEREZ, H.Daniel, Kentfield, CA 94904 (US); WACHTEL, Helmut, 14057 Berlin (DE); LÖSCHMANN, Peter-Andreas, 72379 Hechingen (DE); GRAF, Hermann, 13465 Berlin (DE); HEDGPETH, Joel, San Francisco, CA 94127 (US); SCHMIECHEN, Ralph, 12101 Berlin (DE)
(86) International application number: IB9500332
(87) International publication number: WO95028926

(56) References cited:
- WO-A-92/19594
- J. NEUROBIOLOGY, vol. 54, no. 1-2, 1994 page 198 SOMMER ET AL. 'Tumor-necrosis factor-directed therapy of autoimmune encephalomyelitis by the phosphodiesterase IV inhibitor rolipram'
- ACTA NEUROLOGICA SCANDINAVICA, vol. 88, no. 2, 1993 pages 97-99, NATAF ET AL. 'Pentoxifylline inhibits experimental allergic encephalomyelitis'
- EUR. J. PHARMACOL., vol. 230, no. 1, 5 January 1993 pages 9-14, SCHADE ET AL. 'The specific Type III and IV Phosphodiesterase inhibitor zardaverine suppresses formation of tumor necrosis factor by macrophages'

## Description

### Background of the Invention

Demyelinating diseases are severe afflictions of the brain and spinal cord, involving the destruction of the myelin sheath which surrounds nerve fibers. As a result of demyelination, various neurological symptoms are manifested, including motor impairment, visual loss, and sensory changes. Multiple sclerosis (MS) is the most common of the demyelinating diseases. It is a disease characterized by episodes of focal disorder of the optic nerves, spinal cord, and brain. It is a severe chronic disabling disease with characteristic demyelination in the CNS triggered by probable autoimmune mechanisms in a genetically susceptible population. Typically, it produces recurring episodes of neurologic dysfunction, followed by remission (relapsing-remitting), but it may also be chronic. Although MS is a well-studied disease, its precise cause remains undetermined. One explanation is that an environmental factor which most often acts in childhood activates a specific population of T-cells (with the potential of attacking myelin-associated antigens such as MBP, MAP, MOP or others) which normally is controlled by suppressor-cells. In many MS cases, non-specific stress results in disease exacerbations with opening of the BBB, edema, immigration and activation of T-cells and macrophages and subsequent destruction of oligodendroglia- associated myelin followed by failing attempts at remyelination and finally a glia scar (relapsing - remitting form of MS). These exacerbations which can be visualized by MRI are associated, depending on location, with severe functional disabilities; furthermore, with an increasing number of relapses, the disease (and the antigen being attacked) becomes more generalized and progresses with less clearcut intervals (progressive form of MS, secondary or primary) and increasing and persisting disability which causes (in this mostly young population) impairment of life qualify with loss of employment and independent life (with hospitalization and eventually death).

Until recently, treatments have been empirical and not entirely successful. See Cecil's Textbook of Medicine (Wyngaarden, 1993). Classic non-specific immunosuppressive therapies (including corticosteroids) and cytostatic drugs as a rule have failed to alter this sequence of events and disease progression - possibly because they are poorly tolerated in these patients and also because they inhibit endogenous immunosuppressive mechanisms as well. For a review of treatments, e.g., see *Principles of Neurology*, Fifth Edition, (Adams et al., 1993). Recently non-specific immunomodulatory therapy with interferon-β-1b has been shown to prolong disease-free intervals in patients with beginning relapsing/remitting MS; however, in the great majority of these patients, exacerbations cannot be prevented completely, and also the effect on disability during the first three years of therapy is still small. Corticosteroid therapy relieves some acute symptoms of these exacerbations (probably by reducing edema) but does not affect long term prognosis. However, it appears of utmost importance to suppress all exacerbations with their activation, extension and amplification of autoimmune mechanisms which make the disease uncontrollable and disabling. There remains a need for additional drugs which have an effect on the diseases's severity and progression.

### Summary of the Invention

Recent advance in the clinical, biochemical and imaging technologies enable clinicians to predict and diagnose such exacerbations in a very early stage, and thus, it now becomes possible to combine different therapeutic strategies to achieve a maximal therapeutic effect. Immunomodulatory therapies with non-specif ic mechanisms, e.g., with interferon-β-1b (or specific immunomodulatory drugs such as copolymer I, as well as inducing tolerance to MS antigens) which will never work to 100% as they need to be given before the onset of the disease, but they can reduce to a clear and significant degree the number of clinical exacerbations and, even more, of lesions in the brain. Fortunately, for the target of bringing disease activity to a complete hold, the new diagnostic techniques being developed have the potential to detect the very early beginning of an exacerbation (e.g., increases in γ-interferons, TNF-α, increased number of activated specific T-cells in the blood, new specific MRI and other imaging techniques, and clinical observation). Therefore, strong and efficient drugs are necessary which act not just on some symptoms of the exacerbation but are able to prevent them completely and thus, inhibit disease progression. However, also new therapeutic strategies which aim, e.g., at peripheral T-cell activation, endothelial adhesion, opening of blood-brain barrier and activation of the T-cell-macrophage/microglia interaction with subsequent oligodendrocyte damage and demyelination do affect defense mechanisms not just in the autoimmune condition but, on chronic use, are also damaging vital defense mechanism against exogenous (e.g., bacterial, parasitic or viral infections) as well as against endogenous harmful agents and effects (e.g., tumorigenesis).

An aspect of the present invention is to make possible the combination of therapies with different mechanisms to achieve maximum efficacy which will improve tolerability of therapy (as the effects of interferon-β-1b in MS have been shown to be dose-dependent, higher dosages could be expected to achieve higher and even 100% efficacy, but increasingly severe side effects prevent this type of treatment), and finally, these new combinations of maintenance and anti-exacerbation therapies result in a clearly reduced risk of side effects which can be caused by high-dose and long-term use of these drugs in monotherapy. Indeed when basic maintenance therapy is being combined with the use of these new strategies only when needed (e.g., during or just before an exacerbation) less side effects of both complementary and synergistic therapeutic lines do occur and these forms of therapy can be combined to achieve an optimal clinical result. Also, in this new concept of short-term therapy of exacerbations, different drugs can be combined to prevent further damage and disease progression. Thus, inhibition of synthesis of cytotoxic cyto- and chemokines can be combined with drugs which inhibit their release, and both may be enhanced in efficacy by, e.g., simultaneous inhibition of traffic across the blood-brain barrier, or with other drugs which inhibit - or even reverse - the ultimate tissue damage (e.g., nerve growth factors, calcipotriols, calpain inhibitors, etc.).

| | pathogenic cascade | possible therapeutic intervention |
|---|---|---|
| 1.- | activation of peripheral specific T-cells which are potentially autoreactive to myelin | destroy these T-cells reactivate tolerance by oral antigens, immune globulins? enhance suppressor mechanisms, e.g., interferon-β-1b |
| 2.- | enhanced blood-brain barrier permeability to these T-cells | close blood-brain barrier, e.g., by anti-adhesion molecules such as anti-integrin monoclonal antibodies |
| 3.- | immigration of macrophages, and macrophage-T-cell-interaction and activation | interfere with T-cell receptors, enhance suppressor mechanisms, e.g., by interferon β-1b |
| 4.- | local inflammation and edema | non-specific anti-inflammatory and anti-edema strategies, e.g., corticosteroids |
| 5.- | enhance release of γ-interferons, TNFα and other cytotoxis cytokines | antagonize γ-interferon, TNFα and other cytotoxic cytokines by acting on synthesis, release or other targets (e.g., receptors), e.g., be interferon β-1b |
| 6.- | oligodendrocyte injury with acute myelin breakdown and degradation of axonal lamellae by microglia/macrophages | prevent myelin injury, e.g., with external competing antigens, such as CoP I |
| 7.- | acute conduction block, with functional impairment | act on ion channels, e.g., potassium channel blockers |
| 8.- | chronic de- and remyelination process, functional adaptation and reorganization within the brain | enhance oligoden-drocyte remyelination mechanisms, e.g., by glial growth factors, or Schwann cell implantation |
| 9.- | persisting lesion without or with astrocyte scare and functional impairment, depending on size and location | symptomatic therapies against spasticity, fatigue, urinary problems, etc., therapeutic aids and training of remaining skills |

While many compounds can attack or another of these MS mechanisms and have, as a rule, typical risks and side effects associated with any specific mechanism, our experimental studies have, surprisingly, demonstrated that inhibitors of phosphodiesterase IV, such a rolipram, are very highly effective on human MS cells, as well as, in different animal models of the disease (e.g., EAE, EAN in different species); furthermore, in different human conditions, their safety and efficacy on different biological parameters has already been observed. With these combined mechanisms it is obvious that low dosages can be used which in monotherapy, or even more so in combination with other maintenance therapies, can be used to prevent relapses or reduce or treat exacerbations of MS with very minor side effects (in contrast to other therapies).

Furthermore, at the same or lower (exceptionally also higher) dosages these drugs can be combined with other compounds as described from 1-9 to achieve additive and synergistic therapeutic effects in order to achieve maximum efficacy (and thus inhibition of progression as described previously) with a minimum of side effects.

An aspect of the present invention thus relates to a method of treating or preventing MS comprising administering an effective amount of a combination of a Type IV phosphodiesterase inhibitor (PDE IV inhibitor) as claimed and an anti-inflammatory or immunomodulatory drug for the manufacture of a medicament for treating or preventing MS.

The phosphodiesterase PDE inhibitors suitable for use in this invention are defined in the claims. Cycloadenosine-3',5'-monophosphate (cAMP) PDE type IV (PDE IV) inhibitors according to the modern classification (J.A. Beavo and D.A. Reifsnyder, Trends Pharmacol. Sci. 11; 150-155, 1990) include, but are not limited to compounds disclosed in U.S. 4,193,629, WO 92/02220; U.S. 4,186,129; EP 247 725; U.S. 5,064,854; N.A. Saccamono et al., J. Med. Chem. 34, 291-298, 1991; F.J. Vinick et al., J. Med. Chem. 34, 86-89, 1991; J. A. Lowe et al., J. Med. Chem. 34, 624-628, 1991;. 4-[(3-butoxy-4-methoxyphenyl)methyl]-2-imidazolidinone (RO20-1724); and the pharmaceutically acceptable salts thereof.

Preferred PDE IV inhibitors are racemic and optically active compounds of formula I 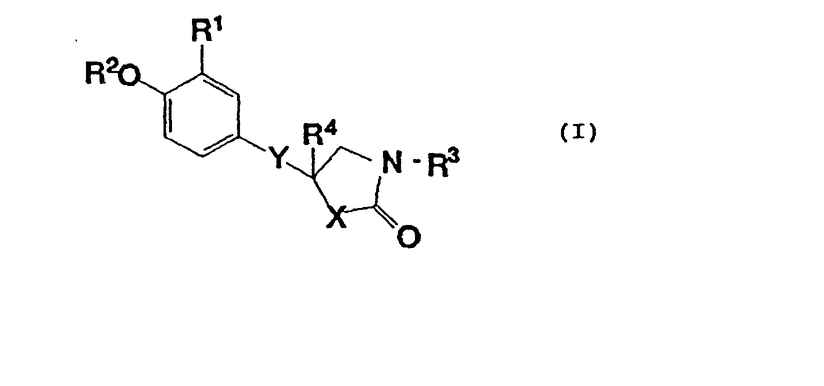 wherein
- R¹: is C₁₋₆-alkyl, a heterocyclic ring, or OR⁵; and
- R⁵: is C₁₋₆-alkyl, C₃₋₇-cycloalkyl, C₂₋₆-alkenyl, C₃₋₇-alkinyl, C₃₋₇-cycloalkyl-C₁₋₂-alkyl, aryl, aralkyl, a heterocyclic ring or C₁₋₆-alkyl substituted by one or more halogen atoms, hydroxy, carboxy, C₁₋₄-alkoxy, C₁₋₄-alkoxycarbonyl, or an optionally alkyl substituted amino group;
- R²: is C₁₋₄-alkyl, C₂₋₄-alkenyl, or C₂₋₄-alkinyl;
- R³: is a hydrogen atom, C₁₋₆-alkyl, aryl, aralkyl, or aryl optionally substituted by one or two methyl groups or C₁₋₆-alkanoyl;
- R⁴: is a hydrogen atom or C₁₋₆-alkyl;
- Y: is a direct bond or a CH₂ group;
- X: is CH₂, CH₂-CH₂, NH, or an oxygen atom; and the pharmaceutically acceptable salts thereof.

Preferred compounds of formula I are those wherein,
- R²: is methyl;
- R³: is a hydrogen atom or C₁₋₆-alkanoyl;
- R¹: is OR₅; R₅ is C₁₋₆-alkyl, C₃₋₇-cycloalkyl, or 3- tetrahydrofuranyl;
- R⁴: is hydrogen or C₁₋₄-alkyl; and
- X: is a CH₂ group or oxygen.

Especially preferred compounds of formula I are those wherein R³ is hydrogen.

Specifically exemplified is 4-(3-(cyclopentyloxy)-4-methoxyphenyl)-2-pyrrolidinone (rolipram).

The term "alkyl" as used herein include straight or branched alkyl radicals, for example, methyl, ethyl, n-propyl, isopropyl, n-butyl, see-butyl, isobutyl, tert-butyl, pentyl, 2-methyl-butyl, 2,2-dimethylpropyl and hexyl.

By the term "alkenyl" as used herein is meant to include, but not limited to vinyl, 1-propenyl, 2-propenyl, 2-propinyl or 3-methyl-2-propenyl.

By the term "cycloalkyl" or "cycloalkyl alkyl" as used herein is meant to include groups of 3-7 carbon atoms, such as cyclopropyl, cyclopropylmethyl, cyclopentyl or cyclohexyl.

By the term "aryl" or "aralkyl" as used herein is meant an aromatic ring or ring system of 6-10 carbon atoms, preferably monocycle, such as phenyl, benzyl, phenethyl or naphthyl.

By the term "heterocyclic ring" as used herein is meant a saturated ring of 5 to 6 members having a single oxygen, sulfur or nitrogen atom, such as, but not limited to 2- and 3-tetrahydropyranyl, 2- and 3-tetrahydrofuranyl, pyrrolidino, 2-and 3-pyrrolidyl, piperidinino, 2-, 3- and 4- piperidyl and the corresponding N-alkyl pyrrolidyl and piperidyl rings, wherein the alkyl is of 1-4 carbon atoms. Also encompassed within the scope of this invention are heterocyclic rings having more than one hetero atom such as morpholino, piperazino or N-alkyl piperazino.

By the term "halo" as used herein is meant all halogens, i.e., chloro, fluoro, bromo and iodo.

The preparation of the compounds of Formula I can be carried out by the procedure outlined in the above-mentioned patents or by U.S. Patent Nos. 4,153,713; 4,186,129; and 5,298,628; WO 86/02268; or EP 0 247 725. Rolipram is 4-[(3-cyclopentyloxy)-4-methoxyphenyl)-2-pyrrolidinone. See, e.g., *Merck Index*, 11th edition, pp. 1312-1313. Rolipram and related compounds can be prepared, e.g., according to U.S. Patent No. 4,193,926.

The anti-inflammatory and immunomodulatory drugs suitable for use in this invention include but are not limited to:
1. interferon derivatives, e.g., betaserone, β-interferon, β-interferon muteins;
2. prostane derivatives, e.g., compounds disclosed in PCT/DE93/0013, e.g., iloprost, cicaprost;
3. glucocorticoids, e.g., cortisol, prednisolone, methylprednisolone, dexamethasone;
4. immunsuppressives, e.g., cyclosporine A, FK-506, methoxsalene, thalidomide, sulfasalazine, azathioprine, methotrexate;
5. lipoxygenase inhibitors, e.g., zileutone, MK-886, WY-50295, SC-45662, SC-41661A, BI-L-357;
6. leukotriene antagonists, e.g., compounds disclosed in DE 4009117; German patent application P 42 42 390.2; WO 9201675; SC-41930; SC-50605; SC-51146; LY 255283 (D.K. Herron et al., FASEB J; 2: Abstr. 4729, 1988); LY 223982 (D.M. Gapinski et al., J. Med. Chem. 33: 2798-2813, 1990); U-75302 and analogs, e.g., described by J. Morris et al., Tetrahedron Lett. 29: 143-146, 1988, C.E. Burgos et al., Tetrahedron Lett. 30: 5081-5084, 1989; B.M. Taylor et al., Prostaglandins 42: 211-224, 1991; compounds disclosed in U.S. 5,019,573; ONO-LB-457 and analogs, e.g., described by K. Kishikawa et al., Adv. Prostagl. Thrombox. Leukotriene Res. 21: 407-410, 1990; M. Konno et al., Adv. Prostagl. Thrombox. Leukotriene Res. 21: 411-414, 1990; WF-11605 and analogs, e.g., disclosed in U.S. 4,963,583; compounds disclosed in WO 9118601, WO 9118879; WO 9118880, and WO 9118883;
7. antiinflammatory substances, e.g., NPC 16570, NPC 17923 described by L. Noronha-Blab. et al., Gastroenterology 102 (Suppl.): A 672, 1992; NPC 15669 and analogs described by R.N. Burch et al., Proc. Nat. Acad. Sci. USA 88: 355-359, 1991; S. Pou et al., Biochem. Pharmacol. 45: 2123-2127, 1993;
8. peptide derivatives, e.g., ACTH and analogs; soluble TNF-receptors; TNF-antibodies; soluble receptors of interleukines, other cytokines, T-cell-proteins; antibodies against receptors of interleukines, other cytokines, T-cell proteins;
9. calcipotriols and their analogues as activators of syntheses of different nerve growth factors, or these growth factors themselves or small peptides thereof which stimulate oligodendrocyte growth (or prevent their apoptosis or destruction) and enhance remyelination.

Our data show that in their effects on human MS cells as well as in different animal models of demyelinating disease (e.g., different EAE models) various of these new drugs can be combined successfully to achieve better protection with less side effects.

By "immunodulatory drugs", it is meant, e.g., agents which act on the immune system, directly or indirectly, e.g., by stimulating or suppressing a cellular activity of a cell in the immune system, e.g., T-cells, B-cells, macrophages, or other APC cells, or by acting upon components outside the immune system which, in turn, stimulate, suppress, or modulate the immune system, e.g., hormones, receptor agonists or antagonists, and neurotransmitters; immunomodulators can be, e.g., immunosuppressants or immunostimulants.

By "anti-inflammatory drugs", it is meant, e.g., agents which treat inflammatory responses, i.e., a tissue reaction to injury, e.g., agents which treat the immune, vascular or lymphatic systems.

Again, in this new combined therapeutic strategy, there are also many steps where the presumed cascade of MS can be attached by existing or future drugs (see.simplified schedule) after the initial specific or non-specific causes, which include impairment of suppressor T-cell effects on myelin-specific autoimmune T-cells.

The present invention also relates to the use of an effective amount of compound of formula I or II alone, preferably Rolipram, a Type IV phosphodiesterase inhibitor for the manufacture of a medicament for treating or preventing MS.

The invention, in one aspect, relates to the use in the manufacture of a medicament of racemates and optically active 4-(polyalkoxyphenyl)-2-pyrrolidones of formula II which are useful in preventing or treating multiple sclerosis: 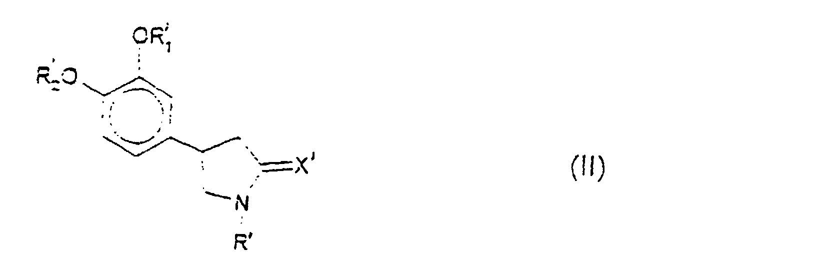 wherein R'₁ and R'₂ each are alike or different and are hydrocarbon of up to 18 carbon atoms with at least one being other than methyl, a heterocyclic ring, or alkyl of 1-5 carbon atoms which is substituted by one or more of halogen atoms, hydroxy, carboxy, alkoxy, alkoxycarbonyl or an amino group; R' is a hydrogen atom, alkyl, aryl or acyl; and X' is an oxygen atom or a sulfur atom.

These compounds and the methods of making them are described, e.g., in U.S. Patent No. 4,193,926 and WO 92/02220.

A preferred compound according to the present invention is Rolipram. Rolipram is 4-[(3-cyclopentyloxy)-4-methoxyphenyl]-2-pyrrolidinone. See, e.g., *Merck Index*, 11th edition, pages 1312-1313. It is commercially available from Schering AG, Berlin, Germany, or may be prepared, e.g., according to Belgian Patent No. 826,923 or U.S. Patent No. 4,193,926. It is useful conventionally as an antidepressant, e.g., U. Schwabe et al., *Mol. Pharmacol.* 12, 900 (1976); H. Wachtel, *Neuropharmacol*. **22,** 267 (1983); H. Wachtel and H. Schneider, *Neuropharmacol*. **25,** 1119 (1986) ; W. Krause and G. Kühne, *Xenobiotica* **18,** 561 (1988). Clinical evaluation of Rolipram for depression is reported in E. Zeller et al., *Pharmacopsychiatry* **17,** 188 (1984). A comparative clinical trial with amitriptyline, *q.v*. in severe depressions is reported in F. Eckmann et al., *Curr. Ther. Res*. **43,** 291 (1988). It is known that, Rolipram, related compounds thereof and Pentoxifylline are TNF inhibitors [D2 = Acta Neurologica Scandinavica, 1993, Vol. 88 (2), p. 97-99; D3 = Eur. J. Pharmacol., 1993, Vol. 230 (1), p. 9-14; and D4 = WO-A-9219594]. Derivatives of Rolipram can also be used according to the invention, i.e. compounds which are structurally related to Rolipram, and are effective in preventing and/or treating MS, e.g., those of formula I.

The present invention also generally relates to the use in the manufacture of a medicament of a Type IV phosphodiesterase inhibitor, preferably a compound of formula I, especially Rolipram, in multiple sclerosis (MS), for preventing, and/or ameliorating the severity, symptoms, and/or periodicity of recurrence of the disease, e.g., lengthening the time period between episodes in which symptoms flare, and/or suppressing the ongoing immune or autoimmune response associated with the disease.

The invention thus makes available the administration of an effective amount of such a compound, e.g., one according to formula I or II, preferably Rolipram, to a patient to prevent or treat MS. The amount of said compound, e.g., Rolipram, administered is an amount which is effective, for example, in preventing or ameliorating the symptoms of the disease or the disease's recurrence, or affecting the ultimate course of the disease, e.g., blocking the inflammatory response in the brain, the appearance of inflammatory lesions, neuronal or neuroglia cell death, and/ or demyelination and the symptoms typically associated with pathogenesis of the disease.

The present invention also provides pharmaceutical compositions comprising a compound according to formula I or II, preferably a Type IV phosphodiesterase inhibitor, preferably Rolipram, or combinations of drugs as described above, which are useful in preventing or treating multiple sclerosis. According to the use , a compound of formula I or II, or drug combinations, can be administered, e.g., in a single dose, in multiple doses, e.g., through-the-skin injection or by sustained release means such as an implanted osmotic pump.

According to the present invention, a pharmaceutical composition of Formulae I and II, or combinations as described above, comprising an effective amount of each compound described can be administered to patients having multiple sclerosis, e.g., multiple sclerosis variants such as Neuromyelitis Optica (Devic's Disease), Diffuse Sclerosis, Transitional Sclerosis, Acute Disseminated Encephalomyelitis, and optic Neuritis.

Symptoms of MS which are prevented or ameliorated or treated include: weakness and/or numbness in one or more limbs; tingling of the extremities and tight band-like sensations around the trunk or limbs; dragging or poor control of one or both legs to spastic or ataxic parepesis; hyperactive tendon reflexes; disappearance of abdominal reflexes; Lhermitte's sign; retrobulbar or optic neuritis; unsteadiness in walking; brain stem symptoms (diplopia, vertigo, vomiting); disorders of micturition; hemiplegia; trigeminal neuralgia; other pain syndromes; nystagmus and ataxia; cerebellar-type ataxia; Charcot's triad; diplopia; bilateral internuclear ophthalmoplegia; myokymia or paralysis of facial muscles; deafness; tinnitus; unformed auditory hallucinations (because of involvement cochlear connections); vertigo and vomiting (vestibular connections); transient facial anesthesia or of trigeminal neuralgia; bladder dysfunction; euphoria; depression; dementia, dull, aching pain in the low back; sharp, burning, poorly localized pains in a limb or both legs and girdle pains; abrupt attacks of neurologic deficit; dysarthria and ataxia; paroxysmal pain and dysesthesia in a limb; flashing lights; paroxysmal itching; and/or tonic seizures, taking the form of flexion (dystonic) spasm of the hand, wrist, and elbow with extension of the lower limb. A patient having MS may have one or more of these symptoms or other clinical manifestations typically associated with MS and one or more can be ameliorated by administrating of compounds according to the present invention.

The administration of Type IV phosphodiesterase inhibitors such as Rolipram, or combinations as claimed, can also block or reduce the physiological and pathogenic deterioration associated with MS, e.g., inflammatory response in the brain and other regions of the nervous system, breakdown or disruption of the blood-brain barrier, appearance of lesions in the brain, tissue destruction, demyelination, autoimmune inflammatory response, acute or chronic inflammatory response, neuronal death, and/or neuroglia death.

The active agents of this invention are useful to treat the different types of MS, including the multifocal, CNS, relapsing and remitting course; the multifocal, CNS, progressive course; the single-site, relapsing and remitting course; and other variants of multiple sclerosis. See, e.g., *Cecil's Textbook of Medicine*, edited by James B. Wyngaarden, 1988.

Effects of the administration of Rolipram and other Type IV phosphodiesterase inhibitors, and combinations of it with other drugs include, e.g., preventing the disease, ameliorating symptoms of the disease, reducing the annual exacerbation rate (i.e., reducing the number of episodes per year), slowing the progression of the disease, or reducing the appearance of brain lesions (e.g., as identified by MRI scan). The episodic recurrence of the mentioned diseases such as MS can be ameliorated, e.g., by decreasing the severity of the symptoms (such as the symptoms described above) associated with the, e.g., MS episode, or by lengthening the time period between the occurrence of episodes, e.g., by days, weeks, months, or years, where the episodes can be characterized by the flare-up and exacerbation of disease symptoms, or preventing or slowing the appearance of brain inflammatory lesions. See, e.g., Adams, R.D., Principles of Neurology, 1993, page 777, for a description of a neurological inflammatory lesion.

By "Type IV phosphodiesterase inhibitor", "specific Type IV phosphodiesterase inhibitor", and similar expressions are meant a selective, i.e., specific, such inhibitor, where the compound binds to or inhibits preferentially the Type IV phosphodiesterase when compared to known types of phosphodiesterase types, e.g., I, II, or III, e.g., whereby the compound has a lower IC₅₀ (more potent) for the Type IV phosphodiesterase, such as where the IC₅₀ is, e.g., 2-fold, 5-fold, 10-fold, 50-fold, or more potent, for the Type IV phosphodiesterase compared to another known type of phosphodiesterase, e.g., I, II, or III. Such selectivity of a compound according to the present invention for a Type IV phosphodiesterase can also be conferred by other means, such as the manner in which it is delivered to its target, e.g., the compound can be associated with an agent which targets it to a specific tissue or cell type having the Type IV phosphodiesterase; the manner in which it interacts with the host's metabolism and/or physiology; or synthesizing PDE inhibitor prodrugs where activation of the PDE inhibitor is accomplished by enzymes present in the desired cells or tissues but absent in others.

The specific inhibition of a Type IV phosphodiesterase can be measured conventionally, e.g., according to the methods described in Reeves et al., *Biochem. J*., 241:535-541, 1977; by macrophage assay, as described, e.g., in Schade et al., Europ. *J. Pharmacol*., 230:9-14, 1993; or WO 93/19068. For a review of phosphodiesterase specificity and how to determine it, see, e.g., Nicholson et al., *Trends Pharmacol. Sci*., 12:19-27 (1991).

The activity of this invention of Type IV phosphodiesterase inhibitors such as Rolipram can be detected, for example, in animals suffering from Experimental Allergic Encephalmyelitis (EAE), an experimental T-lymphocyte initiated disease of the CNS. It can be produced, e.g., in rodents, guinea pigs, rabbits, and primates, by, e.g., immunizing animals with myelin, e.g., from a human brain, and/or corticosteroid administration over a long period of time. It can also be produced by injecting an animal with T-lymphocytes obtained from an animal suffering from EAE.

In particular, the activity can be detected in *Callithrix jacchus* (common marmoset) which has been immunized with myelin, e.g., from a human brain. The *Callithrix jacchus* develops EAE with essentially similar histopathology and neurological symptoms as those at certain stages of the human disease, MS.

The present invention makes available the treatment of MS with a combination of a PDE IV inhibitor as claimed with an interferon derivative, a prostane derivative, a glucocorticoid, an immunosuppressant, a lipoxygenase inhibitor, a leukotriene antagonist, an antiinflammatory substance, a peptide derivative or a calcipotriol or analog thereof.

A preferred combination consists of a PDE IV inhibitor as claimed and an interferon derivative, a prostane derivate or a leukotriene antagonist, e.g., betaseron (interferon β-1b), iloprost, cicaprost, or 5-[(E)-(2S)-2-((1E,3E)-(5S)-5-hydroxy-6,6-trimethylene-9-phenyl-1,3-nonadiene-8-inyl)-cyclohexylidene]-pentanoic acid or esters thereof.

The pharmaceutical compositions according to the present invention are prepared conventionally, comprising substances which are customarily used in pharmaceutical, e.g., see *Remington's Pharmaceutical Sciences*, 18th ed., Mack Publishing Company (1990), including excipients, carriers, adjuvants and buffers. The compositions can be administered, e.g., parenterally, enterally, orally, intramuscularly, topically, subcutaneously, intravenously, by aerosol, intrathecally directly into the cerebral spinal fluid of the CNS, or preferably by sustained release using, e.g., an implanted mini-osmotic pump (e.g., the ALZET pump manufactured by ALZA Corporation, P. O. Box 10950, Palo Alto, CA 94303), or other routes useful to achieve an effect.

Conventional excipients include pharmaceutically acceptable organic or inorganic carrier substances suitable for parenteral, enteral or topical application which do not deleteriously react with the agents. Suitable pharmaceutically acceptable adjuvants include, but are not limited to, water, salt solutions, alcohols, gum arabic, vegetable oils, polyethylene glycols, gelatine, lactose, amylose, magnesium stearate, talc, silicic acid, viscous paraffin, perfume oil, fatty acid monoglycerides and diglycerides, pentaerythritol fatty acid esters, hydroxy-methylcellulose, polyvinyl pyrrolidone, cyclodextrins, etc. The pharmaceutical preparations can be sterilized and, if desired, mixed with stabilizers, wetting agents, emulsifiers, salts for influencing osmotic pressure, buffers, coloring, flavoring and/or aromatic substances, etc., which do not react deleteriously with the active compounds.

For parenteral application, particularly suitable are injectable sterile solutions, preferably oil or aqueous solutions, as well as suspensions, emulsions or implants, including suppositories. Ampoules are convenient unit dosages.

For enteral application, particularly suitable are tablets, dragees, suppositories or capsules having talc and/or a carbohydrate carrier or binder. The carrier may be lactose, corn starch, potato starch or a combination thereof. A syrup or elixir may be used when a sweetened vehicle is employed.

The compositions can also be formulated in an aqueous solution, optionally with the addition of additives customary in galenicals, for example, buffers; electrolytes such as sodium chloride; antioxidants such as ascorbic acid; adjuvants, e.g., methyl cellulose, lactose and mannitol and/or surfactants, e.g., lecithins and Tweens and/or aromatic substances for flavoring, e.g., ethereal oils.

Amounts of Type IV phosphodiesterase inhibitors and drug combinations can be determined routinely based on the information given herein, e.g., using the EAE model. However, any amount which is effective in treating MS can be administered to ameliorate or treat the disease. Dosages are determined conventionally, see, e.g., *Remington's Pharmaceutical Sciences*, 18th ed., Mack Publishing Company (1990). The composition may be administered in a single dose unit or in multiple dosages administered, e.g., twice, three, or four times a day, or by an osmotic pump, which delivers the drug(s) continuously. A Type IV phosphodiesterase inhibitor can be administered at the same time as the anti-inflammatory, immunomodulatory, etc., drug in a single or separate dosage unit, or the drugs can be administered at a different time or, e.g., sequentially.

The exact dose of any component or combination to be administered is determined by the attending clinician and is dependent, e.g., on the potency of the compound administered, the age, weight, condition, and response of the patient.

Generally, PDE IV inhibitors are to be administered alone in amounts of about 0.005 - 2 mg/kg/day, preferably 0.1 -.7 mg/kg/day or 0.5 mg/kg/day, more preferably 0.005 - 0.1 mg/kg/day and the immuno-modulatory or anti-inflammatory, etc., is administered alone in amounts of, e.g., about 0.01 µg/kg/day for a prostacyclin or to about 10 mg/kg/day for a steroid. According to the present invention, the latter can be administered in lower doses than would be expected for purely additive effects, e.g., about 0.0005 to about 0.01 mg/kg/day for a PDE IV inhibitor and about 0.001 µg/kg/day to about 1 mg/kg/day for an immunomodulatory or anti-inflammatory drug.

Since the present invention relates to treatment of MS with a combination of active ingredients wherein said ingredients can be administered separately, the invention also relates to combining separate pharmaceutical composition in kit form. The kit form is particularly advantageous when the separate components are administered in different dosage forms (i.e., oral and parenteral) or are administered at different dosage intervals.

Without further elaboration, it is believed that one skilled in the art can, using the preceding description, utilize the present invention to its fullest extent. The following preferred specific embodiments are, therefore, to be construed as merely illustrative and not limitative of the remainder of the disclosure in any way whatsoever.

In the foregoing and in the following examples, all temperatures are set forth uncorrected in degrees Celsius; and, unless otherwise indicated, all parts and percentages are by weight.

### Brief Description of the Drawings

Various other objects, features, and attendant advantages of the present invention will be more fully appreciated as the same becomes better understood when considered in conjunction with the accompanying drawings, in which like reference characters designate the same or similar parts throughout the several views and wherein:
Figure 1 shows the prevention of Experimental Allergic Encephalomyelitis (EAE) by Rolipram in a marmoset. A, B, and C received Rolipram (10 mg/kg) in DMSO; D and E received an equivalent volume of DMSO. Marmosets immunized with human spinal cord homogenerate received either Rolipram or placebo five days after immunization.
Figure 2 shows the treatment with Rolipram of marmoset having EAE.

### Examples

### EXAMPLE 1

Rolipram was produced by Schering AG (Berlin) and is comprised of (+) and (-) racemates of 4-[(3-cyclopentyloxy)-4-methoxyphenyl)-2-pyrrolidinone. It was dissolved in dimethylsulfoxide (DMSO) at 20 mg/ml.

Human brain white matter homogenate was prepared from autopsy material in complete Freund's adjuvant (CFA) containing *M. tuberculosis* (strain H37 Ra).

Bordetella pertussis vaccine was obtained from the Massachusetts Public Health Department, Biological Laboratories, Boston, Massachusetts.

Zofran was obtained from the University of California Medical Center Pharmacy.

The marmosets were purchased from the New England Regional Primate Research Center and were maintained and cared for in accordance with the guidelines of the Internal Animal Care and Use Committee of the University of California, San Francisco.

Animals were immunized with human brain white matter homogenate (200 mg) in CFA containing 3 mg/ml killed *M*. tuberculosis (H37 Ra strain) by intradermal injection (0.6 ml) over four sites on the doral axilla and inguinal region. On the day of immunization and again 2 days later 10x10¹⁰ inactivated Bordetella pertussis (Bordetella pertussis vaccine) were infused intravenously in 10 ml saline.

On day 5, following immunization, animals were injected subcutaneously in the back of the neck with DMSO (placebo) or with DMSO containing Rolipram to give a dose of 10 mg/kg. Treatment with DMSO or DMSO with Rolipram was preceded 20 min. by an injection of 0.3-0.6 mg/kg Zofran intramuscularly (Odansetron Hydrochloride, Glaxo) to prevent salivation, vomiting, excessive grooming, and head twists. Such treatments were repeated every 48 hours throughout the study.

Animals were observed daily and subjected to a standardized scoring system to record the severity of clinical symptoms:
0. Normal
1. Lethargy, anorexia, weight loss
2. Ataxia, tremor
3. Blindness, paraplegia or hemiplegia
4. Quadraparesis or quadriplegia
5. Moribund

At various times, animals were anesthetized and subjected to MRI.

### Experiment 1: Prevention of EAE by Rolipram Treatment

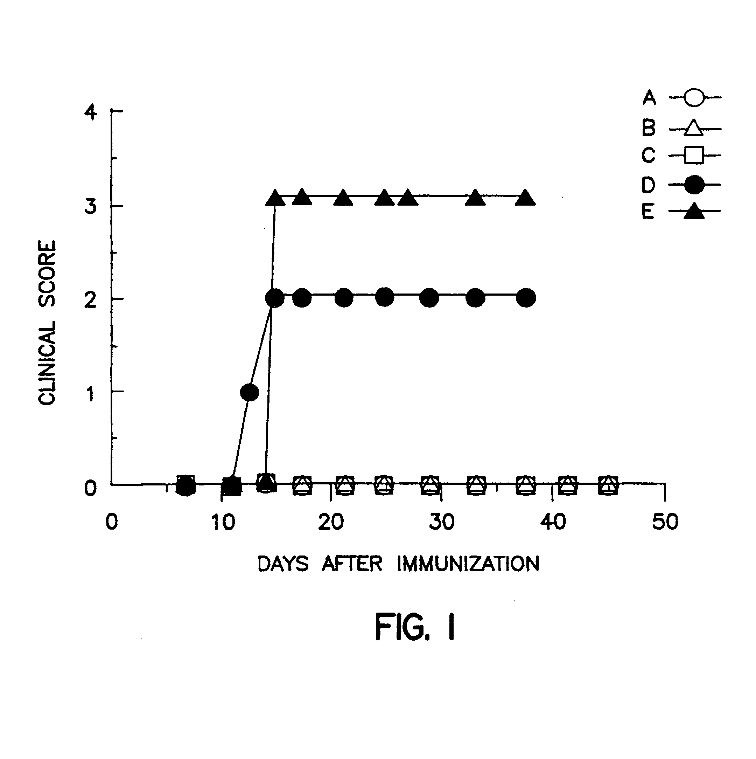

Marmosets were immunized with spinal cord homogenate as described. On day 5, following immunization, three marmosets received Rolipram (10 mg/kg) in DMSO. See Figure 1, A, B, and C. Two marmosets received an equivalent volume of DMSO after the same interval. See Figure 1, D and E. The treatment was repeated every 48 hours.

The animals treated with DMSO (placebo) developed clinical symptoms consistent with EAE 15 days following immunization; see Figure 1, D and E. None of the Rolipram treated animals developed symptoms during the 8 week interval of observation; see Figure 1, A, B, and C.

Magnetic Resonance Imaging (MRI) analysis showed that the two animals with EAE symptoms developed one or more lesions in the brain which "enhanced" with Magnevist (gadolinium, DTPA), indicating an active edematous response consistent with the vascular inflammatory lesions seen in EAE or MS (Alvord, etc.). None of the Rolipram treated animals developed detectable lesions during the 8 week interval of observation.

### Development of EAE After Withdrawal of Rolipram

On day 60, following immunization, the treated animals were removed from treatment and observed for signs of EAE. Two marmosets began to show clinical signs of EAE on day 17, following withdrawal of Rolipram.

### Experiment 2: Treatment of Active EAE with Rolipram

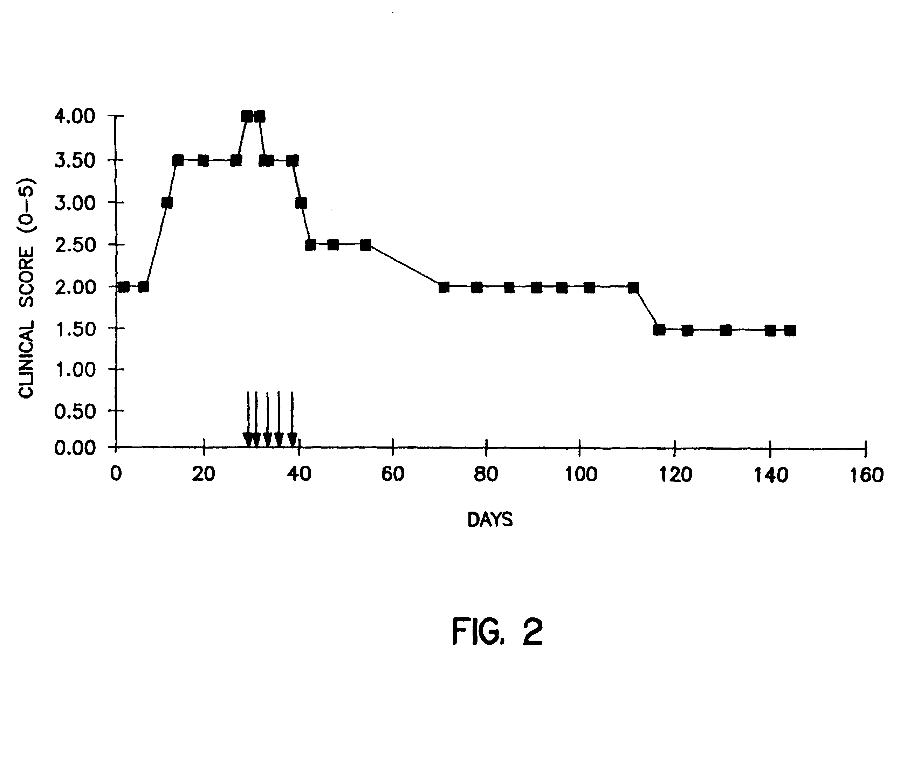

In the previous experiment, it was clearly demonstrated that Rolipram could prevent EAE. It is of interest to determine if Rolipram can also affect active EAE. In this experiment, a marmoset was immunized as previously described, allowed to develop symptoms of chronic EAE and subsequently treated with Rolipram. The animal was treated with escalating doses of Rolipram in DMSO administered as described in the previous experiment. The physical symptoms were monitored as described and MRI analysis was done at times before and after treating (Figure 2).

The animal showed marked improvement on day 10 after initiating treatment. The animal showed MRI improvement on day 14 following initiating Rolipram treatment and, from that time, the condition stabilized with slower improvements.

The results of Experiment 1 indicate that Rolipram treatment blocked the neurological signs of EAE. The MRI results indicate that the inflammatory response was blocked and demyelination did not occur. The untreated control animals developed clear signs of EAE and inflammatory lesions as indicated by MRI analysis. The fact that treated animals developed EAE when removed from treatment showed that the immune response to brain homogenate had occurred sufficiently to initiate the disease; however, some subsequent step in pathogenesis was blocked.

The results of Experiment 2 indicate that Rolipram treatment can inhibit active disease.

### EXAMPLE 2

To assess the ability of rolipram alone or in various combinations as described to modify autoimmune processes, we investigated its influence on TNF production *in vitro* by MBP-specific T-cell lines from MS patients and Lewis rats. MBP is a major candidate antigen in MS, and T-cell-mediated immunity is of crucial importance in its pathogenesis. Similar to EAE, MBP-specific T-cells in humans are often cytotoxic, of Th1 type secreting interferon (IFN)-gamma and TNF/LT, and recognize epitopes that are also encephalitogenic in EAE.

Rolipram selectively inhibited TNF production by human MBP-specific T-cell lines (TCL) in a dose-dependent manner alone and in combination.

Similar results were found using an encephalitogenic CD4+ MBP-specific rat TCL (L1402). TNF/LT (lymphotoxin) production measured in a cytotoxicity bioassay was inhibited in a dose range comparable to the human lines. Moreover, inhibition was stereospecific, with the (-)-enantiomer being 55 times more effective than the (+)- anantiomer. The EC₅₀ of (-)-rolipram, (+)-rolipram, and (-)-rolipram given alone were 20 nM, 280 nM, and 1100 nM, respectively. Previous investigations had shown that inhibition of cAMP PDE by rolipram is stereospecific. In vitro and in vivo binding data in mouse and rat forebrain tissue with ³H-rolipram proved for the (-)-enantiomers a 15-30 times higher affinity than the (+)-enantiomers. In line with these findings, our data strongly suggest that rolipram inhibits TNF/LT production in human and rat autoreactive T-cells by an intracellular CAMP PDE dependent mechanism.

TNF and LT may both be produced by autoactive T-cells. CD4+ cells have been reported to be the major source of TFN in autoimmune insulitis of NOD mice. The cytokine bioassay for TNF/LT detection employed here is sensitive to TNF and LT, but is 200 times more sensitive to the former.

The results of our in vivo findings prompted us to perform treatment experiments in EAE after active immunization (aEAE) and adoptive cell transfer (tEAE) in Lewis rats. When rolipram was administered from day 7 through day 23 in aEAE as monotherapy or in various combinations, the appearance of neurological symptoms was completely prevented. In clinically manifest EAE, treatment was started within 6 hours of the onset of symptoms. In the treated group, disease did only progress moderately, whereas the controls developed severe EAE. None of the rolipram-treated animals developed grade 4 (paraplagia), whereas 4 to 7 animals in the vehicle-treated control group reached this level of impairment in one typical experiment. In tEAE similar effects were observed. Prophylactic treatment resulted in only minor symptoms with a mean maximum score (MMN) of 0.3 ± 0.11 (n = 5) in the treated group, as compared to 2.5 ± 0.25 (n = 5) in the controls (p < 0.01). Treatment after onset of symptoms also lead to a marked reduction in maximum severity (treated group: MMS 0.7 ± 0.10, n = 5; vehicle treated matched controls 2.45 ± 0.56, n = 5, p 0.01).

In order to distinguish further between a long-term prophylactic or a temporary suppressive effect, animals received the drug from the day of active immunization until day 11. With this regimen aEAE in treated animals was delayed by 3.5 days, but disease severity and duration was otherwise similar compared to the controls. This indicates that suppression of EAE and presumably TNF/LT production is temporary, and deletion of autoreactive T-cells by rolipram, as, e.g., in the case of cyclophosphamide, is therefore unlikely.

Histological analysis was performed on selected animals with aEAE. There were only few and mild cellular lesions in the prophylactically treated animals. By contrast, two or three animals that were treated after onset of clinical signs showed cellular infiltrates similar to those in the controls. Previous investigations have shown that inflammatory infiltrates in the central nervous system do not necessarily correlate with the degree of neurological deficit. In a study on EAE, induced by myelin oligodendrocyte glycoprotein (MOG)-specific T-cells, the lack of neurological signs was ascribed to a decrease of macrophages and parenchymal inflammation, whereas perivascular inflammation and the synthesis of TNF, IFN-gamma, and interleukin-6 was clearly present. In our system, however, the timely onset of paralysis and the morphologically similar appearance of infiltrates in some of the treated animals argues against such a phenomenon. We propose that during rolipram treatment of clinically manifest EAE, suppression of local TNF production in addition to its other effects is crucial regardless of the discrepancy between the histological and clinical scores. Using in-situ hybridization, it was shown recently that the present of TNF expressing cells in the CNS correlates well with the clinical signs in EAE. Using this approach it should be possible to identify the rolipram-sensitive cell types in EAE and MS lesions - inflammatory cells, glial cells, or both.

In contrast, rolipram by its many effects is expected to be of great therapeutic use in MS and other patients, especially in appropriate combinations, as shown by these and other experiments and as indicated by the considerations already discussed.

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the disclosure thereof, can make various changes and modifications of the invention to adapt it to various usages and conditions as defined by the claims.

## Claims

1. The use of a PDE IV inhibitor according to formula I 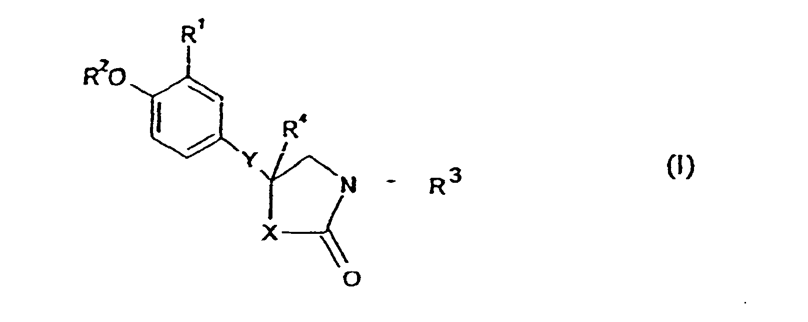 wherein
R¹ is C₁₋₆-alkyl, a heterocyclic ring, or OR⁵; and
R⁵ is C₁₋₆-alkyl, C₃₋₇-cycloalkyl, C₂₋₆-alkenyl, C₃₋₇-alkinyl, C₃₋₇-cycloalkyl-C₁₋₂- alkyl, aryl, aralkyl, a heterocyclic ring or C₁₋₆-alkyl substituted by one or more halogen atoms, hydroxy, carboxy, C₁₋₄-alkoxy, C₁₋₄-alkoxycarbonyl, or an optionally alkyl substituted amino group;
R² is C₁₋₄-alkyl, C₂₋₄-alkenyl, or C₂₋₄-alkinyl;
R³ is a hydrogen atom, C₁₋₆-alkyl, aryl, aralkyl, or aryl optionally substituted by one or two methyl groups or C₁₋₆-alkanoyl;
R⁴ is a hydrogen atom or C₁₋₆-alkyl;
Y is a direct bond or a CH₂ group;
X is CH₂, CH₂-CH₂, NH, or an oxygen atom; and pharmaceutically acceptable salts thereof and an anti-inflammatory or immunomodulatory drug for the manufacture of a medicament for treating or preventing multiple sclerosis.

2. The use according to claim 1, wherein R² is methyl; R³ is hydrogen or C₁₋₆-alkanoyl; R¹ is OR⁵; R⁵ is C₁₋₆-alkyl, C₃₋₇-cycloalkyl or 3-tetrahydrofuranyl; R⁴ is hydrogen or C₁₋₄-alkyl; and X is a CH₂ group or oxygen.

3. The use according to claim 2 wherein R³ is hydrogen.

4. The use according to claim 1, wherein said compound is 4-[(3-cyclo-pentyloxy)-4-methoxyphenyl]-2-pyrrolidinone.

5. The use according to anyone of the claims 22 or 23 wherein the interferon derivative is interferon-β.

6. The use according to claim 5, wherein the interferon β is interferon-β-1b.

7. The use of a PDE IV inhibitor according to formula II 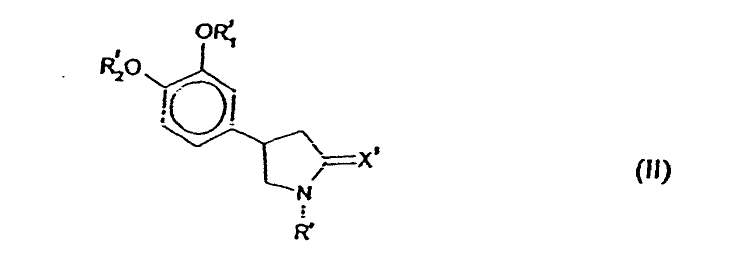 wherein:
R'₁ and R'₂ each are alike or different and are C₁₋₁₈-alkyl with at least one being other than methyl, a heterocyclic ring, or C₁₋₅-alkyl substituted by one or more of halogen atoms, hydroxy, carboxy, alkoxy, alkoxycarbonyl or an amino group;
R' is a hydrogen atom, alkyl, aryl or acyl; and X' is an oxygen atom or a sulfur atom for the manufacture of a medicament for preventing or treating multiple sclerosis.

8. The use according to claim 7, wherein said compound is 4-[(3-cyclo-pentyloxy)-4-methoxyphenyl]-2-pyrrolidinone.

9. The use according to claim 7, wherein one of R'₁ and R'₂ is methyl, and the other is C₃₋₇-cycloalkyl.

10. The use of a PDE IV inhibitor according to formula I 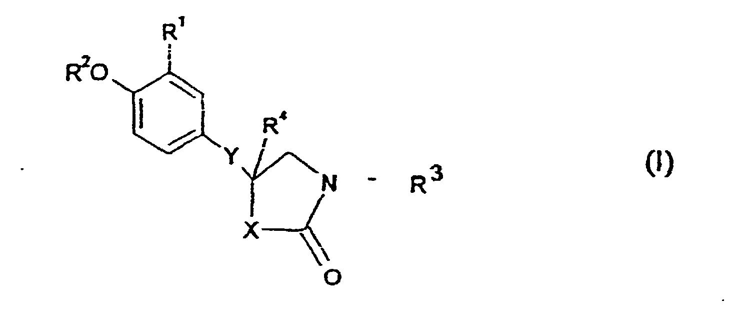 wherein
R¹ is C₁₋₆-alkyl, a heterocyclic ring, or OR⁵; and
R⁵ is C₁₋₆-alkyl, C₃₋₇-cycloalkyl, C₂₋₆-alkenyl, C₃₋₇-alkinyl, C₃₋₇-cycloalkyl-C₁₋₂- alkyl, aryl, aralkyl, a heterocyclic ring or C₁₋₆-alkyl substituted by one or more halogen atoms, hydroxy, carboxy, C₁₋₄-alkoxy, C₁₋₄-alkoxycarbonyl, or an optionally alkyl substituted amino group;
R² is C₁₋₄-alkyl, C₂₋₄-alkenyl, or C₂₋₄-alkinyl;
R³ is a hydrogen atom, C₁₋₆-alkyl, aryl, aralkyl, or aryl optionally substituted by one or two methyl groups or C₁₋₆-alkanoyl;
R⁴ is a hydrogen atom or C₁₋₆-alkyl;
Y is a direct bond or a CH₂ group;
X is CH₂, CH₂-CH₂, NH, or an oxygen atom; and pharmaceuticalty acceptable salts thereof for the manufacture of a medicament for treating or preventing multiple sclerosis.

11. The use according to claim 10, wherein R² is methyl; R³ is hydrogen or C₁₋₆-alkanoyl; R¹ is OR⁵; R⁵ is C₁₋₆-alkyl, C₃₋₇-cycloalkyl or 3-tetrahydrofuranyl; R⁴ is hydrogen or C₁₋₄-alkyl; and X is a CH₂ group or oxygen.

12. The use according to anyone of the claims 1 - 10, wherein the time between or the severity of symptoms of episodic recurrences of multiple sclerosis is ameliorated.

13. The use according to anyone of the claims 1 - 10, wherein an inflammatory lesion associated with said multiple sclerosis is prevented or treated.

14. The use according to anyone of the claims 1 - 10, wherein the appearance of an inflammatory lesion associated with said multiple sclerosis is slowed.

15. The use according to anyone of the claims 1 - 10, wherein the multiple sclerosis is neuromyelitis optica, diffuse sclerosis, transitional sclerosis, acute disseminated encephalomyelitis or optic neuritis.

16. A pharmaceutical composition comprising a PDE IV inhibitor according to formula I 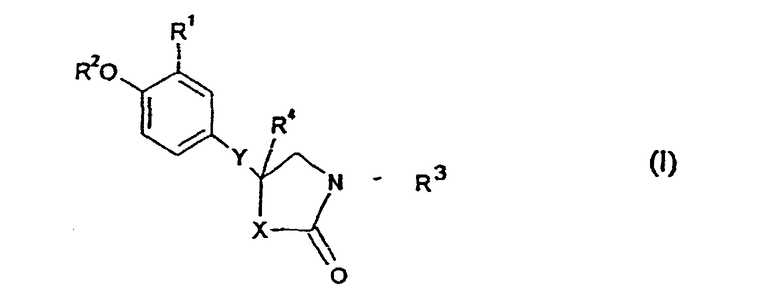 wherein
R¹ is C₁₋₆-alkyl, a heterocyclic ring, or OR⁵; and .
R⁵ is C₁₋₆-alkyl, C₃₋₇-cycloalkyl, C₂₋₆-alkenyl, C₃₋₇-alkinyl, C₃₋₇-cycloalkyl-C₁₋₂- alkyl, aryl, aralkyl, a heterocyclic ring or C₁₋₆-alkyl substituted by one or more halogen atoms, hydroxy, carboxy, C₁₋₄-alkoxy, C₁₋₄-alkoxycarbonyl, or an optionally alkyl substituted amino group;
R² is C₁₋₄-alkyl, C₂₋₄-alkenyl, or C₂₋₄-alkinyl;
R³ is a hydrogen atom, C₁₋₆-alkyl, aryl, aralkyl, or aryl optionally substituted by one or two methyl groups or C₁₋₆-alkanoyl;
R⁴ is a hydrogen atom or C₁₋₆-alkyl;
Y is a direct bond or a CH₂- group;
X is CH₂, CH₂-CH₂, NH, or an oxygen atom; and pharmaceutically acceptable salts thereof and an interferon derivative.

17. A pharmaceutical composition according to claim 16, wherein R² is methyl; R³ is hydrogen or C₁₋₆-alkanoyl; R¹ is OR⁵; R⁵ is C₁₋₆-alkyl, C₃₋₇-cycloalkyl or 3-tetrahydrofuranyl; R⁴ is hydrogen or C₁₋₄-alkyl; and X is a CH₂ group or oxygen.

18. A pharmaceutical composition according to claim 16, wherein said compound is 4-[(3-cyclopentyloxy)-4-methoxyphenyl]-2-pyrrolidinone.

19. A pharmaceutical composition according to anyone of the claims 16 - 18, wherein the interferon derivative is interferon-β.

20. A pharmaceutical composition according to claim 19 wherein the interferon-β is interferon-β-1b.

21. The use according to anyone of the claims 1 - 10 wherein the multiple sclerosis is the multifocal CNS relapsing and remitting course, the multifocal CNS progressive course or the single-site relapsing and remitting course.

22. The use according to claim 1, wherein the anti-inflammatory or immunomodulatory drug is selected from the group consisting of an interferon derivative, a prostane derivative, a glucocorticoid, an immunosuppressant, a lipoxygenase inhibitor, a leukotriene antagonist, an anti-inflammatory substance, a drug peptide derivative or a calcipotriol or analogue thereof.

23. The use according to claim 1 or 22, wherein the drug is an interferon derivative.

## Patentansprüche

1. Verwendung eines PDE IV-Inhibitors gemäß Formel I 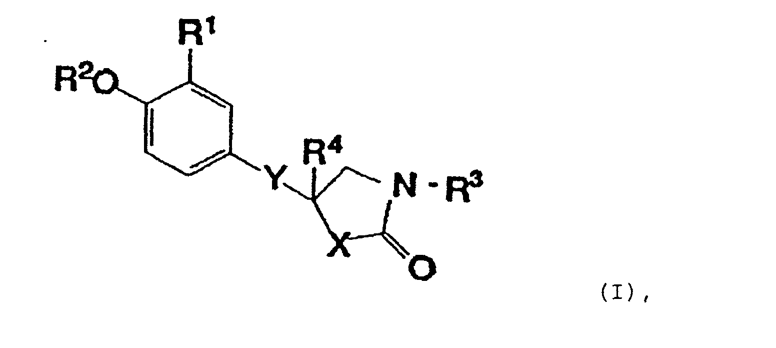 worin
R¹ C₁- bis C₆-Alkyl, ein heterocyclischer Ring oder OR⁵ ist; und
R⁵ C₁- bis C₆-Alkyl, C₃- bis C₇-Cycloalkyl, C₂- bis C₆-Alkenyl, C₃- bis C₇-Alkinyl, C₃- bis C₇-Cycloalkyl-C₁- bis C₂-alkyl, Aryl, Aralkyl, ein heterocyclischer Ring oder C₁- bis C₆-Alkyl ist, das durch ein oder mehrere Halogenatome, Hydroxy, Carboxy, C₁- bis C₄-Alkoxy, C₁- bis C₄-Alkoxycarbonyl oder eine gegebenenfalls alkylsubstituiertes Aminogruppe substituiert ist;
R² C₁- bis C₄-Alkyl, C₂- bis C₄-Alkenyl oder C₂- bis C₄-Alkinyl ist;
R³ ein Wasserstoffatom, C₁- bis C₆-Alkyl, Aryl, Aralkyl oder Aryl, das gegebenenfalls mit einer oder zwei Methylgruppen substituiert ist, oder C₁bis C₆-Alkanoyl ist;
R⁴ ein Wasserstoffatom oder C₁- bis C₆-Alkyl ist;
Y eine direkte Bindung oder eine CH₂-Gruppe ist; X CH₂, CH₂-CH₂, NH oder ein Sauerstoffatom ist; und pharmazeutisch annehmbaren Salze davon, und eines entzündungshemmenden oder immunmodulierenden Arzneimittels zur Herstellung eines Medikaments zur Behandlung oder Verhinderung von multipler Sklerose.

2. Verwendung nach Anspruch 1, bei der R² Methyl ist; R³ Wasserstoff oder C₁- bis C₆-Alkanoyl ist; R¹ OR⁵ ist; R⁵ C₁- bis C₆-Alkyl, C₃- bis C₇-Cycloalkyl oder 3-Tetrahydrofuranyl ist; R⁴ Wasserstoff oder C₁- bis C₄-Alkyl ist; und X eine CH₂-Gruppe oder Sauerstoff ist.

3. Verwendung nach Anspruch 2, bei der R³ Wasserstoff ist.

4. Verwendung nach Anspruch 1, bei der die Verbindung 4-[(3-Cyclopentyloxy)-4-methoxyphenyl]-2-pyrrolidinon ist.

5. Verwendung nach einem der Ansprüche 22 oder 23, bei der das Interferonderivat Interferon-β ist.

6. Verwendung nach Anspruch 5, bei der das Interferon-β Interferon-β-1b ist.

7. Verwendung eines PDE IV-Inhibitors gemäß Formel II 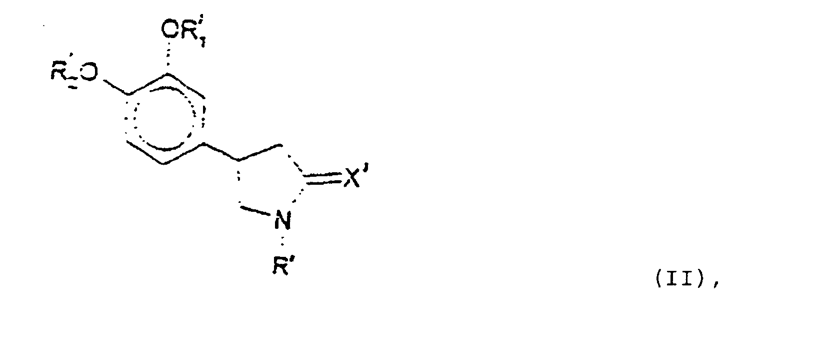 worin
R'₁ und R'₂ jeweils gleich oder unterschiedlich und C₁- bis C₁₈-Alkyl, wobei mindestens eines von Methyl verschieden ist, ein heterocyclischer Ring oder C₁- bis C₅-Alkyl sind, das mit einem oder mehreren von Halogenatomen, Hydroxy, Carboxy, Alkoxy, Alkoxycarbonyl oder einer Aminogruppe substituiert ist;
R' ein Wasserstoffatom, Alkyl, Aryl oder Acyl ist; und X' ein Sauerstoffatom oder Schwefelatom ist;
zur Herstellung eines Medikaments zur Verhinderung oder Behandlung der multiplen Sklerose.

8. Verwendung nach Anspruch 7, bei der die Verbindung 4-[(3-Cyclopentyloxy)-4-methoxyphenyl]-2-pyrrolidinon ist.

9. Verwendung nach Anspruch 7, bei der einer von R'₁ und R'₂ Methyl ist und der andere C₃- bis C₇-Cycloalkyl ist.

10. Verwendung eines PDE IV-Inhibitors gemäß Formel I 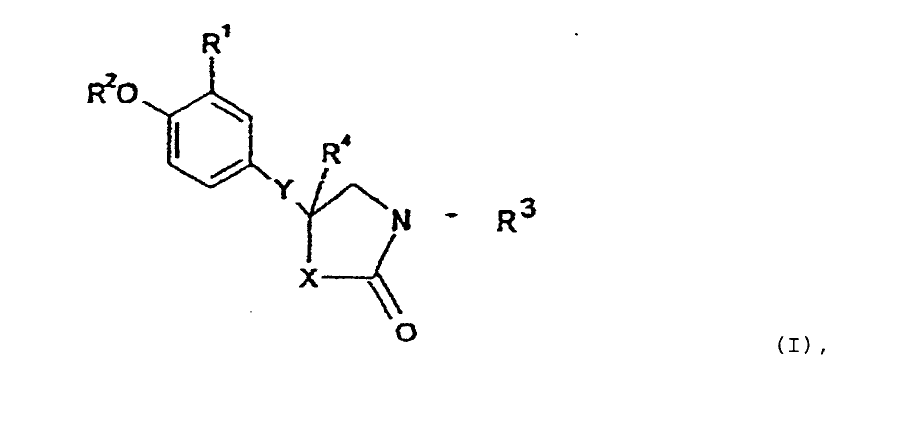 worin
R¹ C₁- bis C₆-Alkyl, ein heterocyclischer Ring oder OR⁵ ist; und
R⁵ C₁- bis C₆-Alkyl, C₃- bis C₇-Cycloalkyl, C₂- bis C₆-Alkenyl, C₃- bis C₇-Alkinyl, C₃- bis C₇-Cycloalkyl-C₁- bis C₂-alkyl, Aryl, Aralkyl, ein heterocyclischer Ring oder C₁- bis C₆-Alkyl ist, das durch ein oder mehrere Halogenatome, Hydroxy, Carboxy, C₁- bis C₄-Alkoxy, C₁- bis C₄-Alkoxycarbonyl oder eine gegebenenfalls alkylsubstituiertes Aminogruppe substituiert ist;
R² C₁- bis C₄-Alkyl, C₂- bis C₄-Alkenyl oder C₂- bis C₄-Alkinyl ist;
R³ ein Wasserstoffatom, C₁- bis C₆-Alkyl, Aryl, Aralkyl oder Aryl, das gegebenenfalls mit einer oder zwei Methylgruppen substituiert ist, oder C₁bis C₆-Alkanoyl ist;
R⁴ ein Wasserstoffatom oder C₁- bis C₆-Alkyl ist;
Y eine direkte Bindung oder eine CH₂-Gruppe ist;
X CH₂, CH₂-CH₂, NH oder ein Sauerstoffatom ist; und
pharmazeutisch annehmbarer Salze davon,
zur Herstellung eines Medikaments zur Behandlung oder Verhinderung von multipler Sklerose.

11. Verwendung nach Anspruch 10, bei der R² Methyl ist; R³ Wasserstoff oder C₁- bis C₆-Alkanoyl ist; R¹ OR⁵ ist; R⁵ C₁- bis C₆-Alkyl, C₃- bis C₇-Cycloalkyl oder 3-Tetrahydrofuranyl ist; R⁴ Wasserstoff oder C₁- bis C₄-Alkyl ist; und X eine CH₂-Gruppe oder Sauerstoff ist.

12. Verwendung nach einem der Ansprüche 1 bis 10, bei der die Zeit zwischen den Schüben oder die Schwere der Symptome der Schübe der multiplen Sklerose verlängert bzw. gelindert wird.

13. Verwendung nach einem der Ansprüche 1 bis 10, bei der eine mit der multiplen Sklerose verbundene entzündliche Läsion verhindert oder behandelt wird.

14. Verwendung nach einem der Ansprüche 1 bis 10, bei der das Auftreten einer mit der multiplen Sklerose verbundenen entzündlichen Läsion verzögert wird.

15. Verwendung nach einem der Ansprüche 1 bis 10, bei der die multiple Sklerose Neuromyelitis optica, diffuse Sklerose, transitorische Sklerose, Encephalomyelitis acuta disseminata oder Neuritis optica ist.

16. Pharmazeutische Zusammensetzung, die einen PDE IV-Inhibitor gemäß Formel I 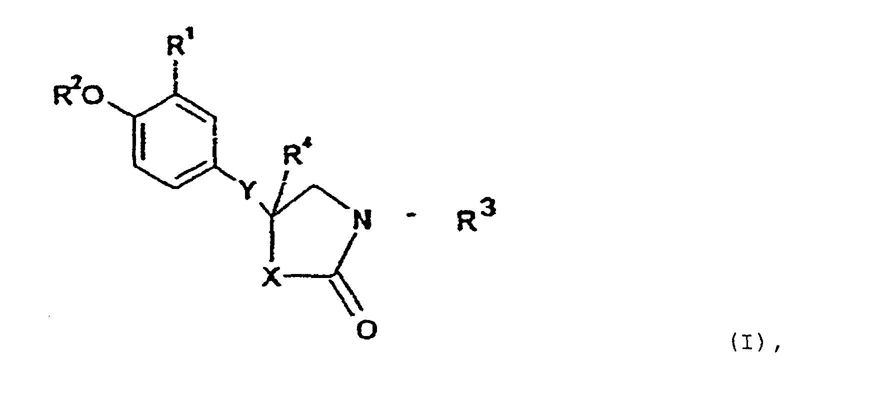 worin
R¹ C₁- bis C₆-Alkyl, ein heterocyclischer Ring oder OR⁵ ist; und
R⁵ C₁- bis C₆-Alkyl, C₃- bis C₇-Cycloalkyl, C₂bis C₆-Alkenyl, C₃- bis C₇-Alkinyl, C₃- bis C₇-Cycloalkyl-C₁- bis C₂-alkyl, Aryl, Aralkyl, ein heterocyclischer Ring oder C₁- bis C₅-Alkyl ist, das durch ein oder mehrere Halogenatome, Hydroxy, Carboxy, C₁- bis C₄-Alkoxy, C₁- bis C₄-Alkoxycarbonyl oder eine gegebenenfalls alkylsubstituiertes Aminogruppe substituiert ist;
R² C₁- bis C₄-Alkyl, C₂- bis C₄-Alkenyl oder C₂bis C₄-Alkinyl ist;
R³ ein Wasserstoffatom, C₁- bis C₆-Alkyl, Aryl, Aralkyl oder Aryl, das gegebenenfalls mit einer oder zwei Methylgruppen substituiert ist, oder C₁- bis C₆-Alkanoyl ist;
R⁴ ein Wasserstoffatom oder C₁- bis C₆-Alkyl ist;
Y eine direkte Bindung oder eine CH₂-Gruppe ist;
X CH₂, CH₂-CH₂, NH oder ein Sauerstoffatom ist;
und pharmazeutisch annehmbare Salze davon, und
ein Interferonderivat umfasst.

17. Pharmazeutische Zusammensetzung nach Anspruch 16, bei der R² Methyl ist; R³ Wasserstoff oder C₁- bis C₆-Alkanoyl ist; R¹ OR⁵ ist; R⁵ C₁-bis C₆-Alkyl, C₃bis C₇-Cycloalkyl oder 3-Tetrahydrofuranyl ist; R⁴ Wasserstoff oder C₁- bis C₄-Alkyl ist; und X eine CH₂-Gruppe oder Sauerstoff ist.

18. Pharmazeutische Zusammensetzung nach Anspruch 16, bei der die Verbindung 4-[(3-Cyclopentyloxy)-4-methoxyphenyl]-2-pyrrolidinon ist.

19. Pharmazeutische Zusammensetzung nach einem der Ansprüche 16 bis 18, bei der das Interferonderivat Interferon-β ist.

20. Pharmazeutische Zusammensetzung nach Anspruch 19, bei der das Interferon-β Interferon-β-1b ist.

21. Verwendung nach einem der Ansprüche 1 bis 10, bei der die multiple Sklerose den multifokalen, schubförmigen ZNS-Verlauf, den multifokalen, progredienten Verlauf oder den Einzelstellen betreffenden, schubförmigen Verlauf aufweist.

22. Verwendung nach Anspruch 1, bei der das entzündungshemmende oder immunmodulierende Arzneimittel ausgewählt ist aus der Gruppe bestehend aus einem Interferonderivat, einem Prostanderivat, einem Glukokortikoid, einem Immunsuppressivum, einem Lipoxygenase-Inhibitor, einem Leukotrien-Antagonisten, einer entzündungshemmenden Substanz, einem Arzneipeptidderivat oder einem Calcipotriol oder Analogon davon.

23. Verwendung nach Anspruch 1 oder 22, bei dem das Arzneimittel ein Interferonderivat ist.

## Revendications

1. Utilisation d'un inhibiteur de PDE IV selon la formule I 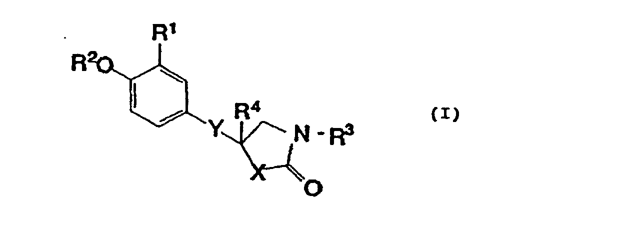 dans laquelle
R¹ est un alkyle en C₁₋₆, un noyau hétérocyclique ou OR⁵ ; et
R⁵ est un alkyle en C₁₋₆, un cycloalkyle en C₃₋₇, un alcényle en C₂₋₆, un alcynyle en C₃₋₇, un C₃₋₇-cycloalkyl-C₁₋₂-alkyle, un aryle, un aralkyle, un noyau hétérocyclique ou un alkyle en C₁₋₆ substitué par un ou plusieurs atomes d'halogène, un hydroxy, un carboxy, un alcoxy en C₁₋₄, un C₁₋₄-alcoxycarbonyle ou un groupe amino éventuellement substitué par un alkyle ;
R² est un alkyle en C₁₋₄, un alcényle en C₂₋₄ ou un alcynyle en C₂₋₄ ;
R³ est un atome d'hydrogène, un alkyle en C₁₋₆, un aryle, un aralkyle ou un aryle éventuellement substitué par un ou deux groupes méthyle ou un alcanoyle en C₁₋₆ ;
R⁴ est un atome d'hydrogène ou un alkyle en C₁₋₆ ;
Y est une liaison directe ou un groupe CH₂ ;
X est CH₂, CH₂-CH₂, NH ou un atome d'oxygène ; et les sels pharmaceutiquement acceptables de ceux-ci et d'un médicament anti-inflammatoire ou immunomodulateur pour la fabrication d'un médicament pour le traitement ou la prévention de la sclérose en plaques.

2. Utilisation selon la revendication 1, dans laquelle R² est un méthyle ; R³ est un hydrogène ou un alcanoyle en C₁₋₆ ; R¹ est OR⁵ ; R⁵ est un alkyle en C₁₋₆, un cycloalkyle en C₃₋₇ ou un 3-tétrahydrofuranyle ; R⁴ est un hydrogène ou un alkyle en C₁₋₄ ; et X est un groupe CH₂ ou un oxygène.

3. Utilisation selon la revendication 2, dans laquelle R³ est un hydrogène.

4. Utilisation selon la revendication 1, dans laquelle ledit composé est la 4-[(3-cyclopentyloxy)-4-méthoxyphényl]-2-pyrrolidinone.

5. Utilisation suivant l'une quelconque des revendications 22 ou 23, dans laquelle le dérivé de l'interféron est l'interféron β.

6. Utilisation selon la revendication 5, dans laquelle l'interféron β est l'interféron β-1b.

7. Utilisation d'un inhibiteur de PDE IV selon la formule II 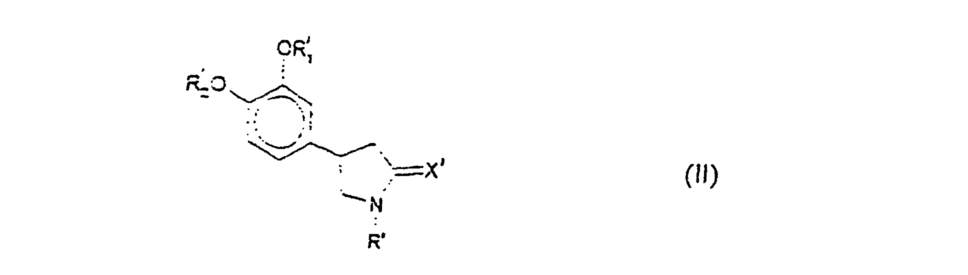 dans laquelle :
R'₁ et R'₂ sont chacun identiques ou différents et sont un alkyle en C₁₋₁₈, au moins l'un étant autre qu'un méthyle, un noyau hétérocyclique, un alkyle en C₁₋₅ substitué par un ou plusieurs parmi des atomes d'halogène, un groupe hydroxy, carboxy, alcoxy, alcoxycarbonyle ou amino ;
R' est un atome d'hydrogène, un alkyle, un aryle
ou un acyle ; et X' est un atome d'oxygène ou un atome de soufre, pour la fabrication d'un médicament destiné à la prévention ou au traitement de la sclérose en plaques.

8. Utilisation selon la revendication 7, dans laquelle ledit composé est la 4-[(3-cyclopentyloxy)-4-méthoxyphényl]-2-pyrrolidinone.

9. Utilisation selon la revendication 7, dans laquelle l'un parmi R'₁ et R'₂ est un méthyle, et l'autre est un cycloalkyle en C₃₋₇.

10. Utilisation d'un inhibiteur de PDE IV selon la formule I 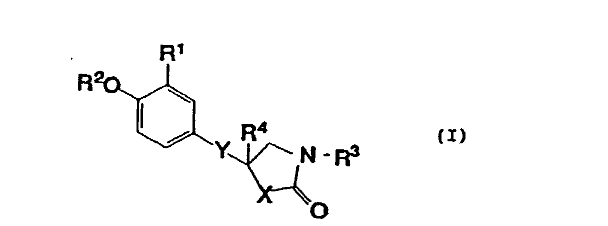 dans laquelle
R¹ est un alkyle en C₁₋₆, un noyau hétérocyclique ou OR⁵ ; et
R⁵ est un alkyle en C₁₋₆, un cycloalkyle en C₃₋₇, un alcényle en C₂₋₆, un alcynyle en C₃₋₇, un C₃₋₇-cycloalkyl-C₁₋₂-alkyle, un aryle, un aralkyle, un noyau hétérocyclique ou un alkyle en C₁₋₆ substitué par un ou plusieurs atomes d'halogène, un hydroxy, un carboxy, un alcoxy en C₁₋₄, un C₁₋₄-alcoxycarbonyle ou un groupe amino éventuellement substitué par un alkyle ;
R² est un alkyle en C₁₋₄, un alcényle en C₂₋₄ ou un alcynyle en C₂₋₄ ;
R³ est un atome d'hydrogène, un alkyle en C₁₋₆, un aryle, un aralkyle ou un aryle éventuellement substitué par un ou deux groupes méthyle ou un alcanoyle en C₁₋₆ ;
R⁴ est un atome d'hydrogène ou un alkyle en C₁₋₆ ;
Y est une liaison directe ou un groupe CH₂ ;
X est CH₂, CH₂-CH₂, NH ou un atome d'oxygène ; et des sels pharmaceutiquement acceptables de celui-ci pour la fabrication d'un médicament destiné au traitement ou à la prévention de la sclérose en plaques.

11. Utilisation selon la revendication 10, dans laquelle R² est un méthyle ; R³ est un hydrogène ou un alcanoyle en C₁₋₆ ; R¹ est OR⁵ ; R⁵ est un alkyle en C₁₋₆, un cycloalkyle en C₃₋₇ ou un 3-tétrahydrofuranyle ; R⁴ est un hydrogène ou un alkyle en C₁₋₄ ; et X est un groupe CH₂ ou un oxygène.

12. Utilisation selon l'une quelconque des revendications 1 à 10, dans laquelle la période entre ou la gravité de symptômes des récidives épisodiques de la sclérose en plaques est améliorée.

13. Utilisation selon l'une quelconque des revendications 1 à 10, dans laquelle une lésion inflammatoire associée à ladite sclérose en plaques est prévenue ou traitée.

14. Utilisation selon l'une quelconque des revendications 1 à 10, dans laquelle l'apparition d'une lésion inflammatoire associée à ladite sclérose en plaques est ralentie.

15. Utilisation selon l'une quelconque des revendications 1 à 10, dans laquelle la sclérose en plaques est la neuromyélite optique, la sclérose diffuse, la sclérose transitionnelle, l'encéphalomyélite aiguë disséminée ou la névrite optique.

16. Composition pharmaceutique comprenant un inhibiteur de PDE IV selon la formule I 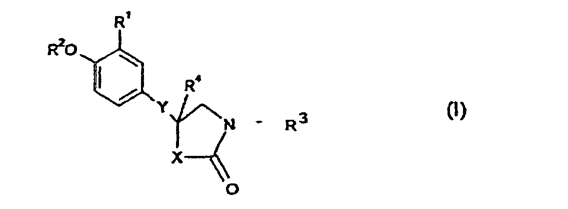 dans laquelle
R¹ est un alkyle en C₁₋₆, un noyau hétérocyclique ou OR⁵ ; et
R⁵ est un alkyle en C₁₋₆, un cycloalkyle en C₃₋₇, un alcényle en C₂₋₆, un alcynyle en C₃₋₇, un C₃₋₇-cycloalkyl-C₁₋₂-alkyle, un aryle, un aralkyle, un noyau hétérocyclique ou un alkyle en C₁₋₆ substitué par un ou plusieurs atomes d'halogène, un hydroxy, un carboxy, un alcoxy en C₁₋₄, un C₁₋₄-alcoxycarbonyle ou un groupe amino éventuellement substitué par un alkyle ;
R² est un alkyle en C₁₋₄, un alcényle en C₂₋₄ ou un alcynyle en C₂₋₄ ;
R³ est un atome d'hydrogène, un alkyle en C₁₋₆, un aryle, un aralkyle ou un aryle éventuellement substitué par un ou deux groupes méthyle ou un alcanoyle en C₁₋₆ ;
R⁴ est un atome d'hydrogène ou un alkyle en C₁₋₆ ;
Y est une liaison directe ou un groupe CH₂ ;
X est CH₂, CH₂-CH₂, NH ou un atome d'oxygène ; et des sels pharmaceutiquement acceptables de celui-ci et un dérivé de l'interféron.

17. Composition pharmaceutique selon la revendication 16, dans laquelle R² est un méthyle ; R³ est un hydrogène ou un alcanoyle en C₁₋₆ ; R¹ est OR⁵ ; R⁵ est un alkyle en C₁₋₆, un cycloalkyle en C₃₋₇ ou un 3-tétrahydrofuranyle ; R⁴ est un hydrogène ou un alkyle en C₁₋₄ ; et X est un groupe CH₂ ou un oxygène.

18. Composition pharmaceutique selon la revendication 16, dans laquelle ledit composé est la 4-[(3-cyclopentyloxy)-4-méthoxyphényl]-2-pyrrolidinone.

19. Composition pharmaceutique selon l'une quelconque des revendications 16 à 18, dans laquelle le dérivé de l'interféron est l'interféron β.

20. Composition pharmaceutique selon la revendication 19, dans laquelle l'interféron β est l'interféron β-1b.

21. Utilisation selon l'une quelconque des revendications 1 à 10, dans laquelle la sclérose en plaques est l'évolution récidivante et rémittente multifocale du CNS, l'évolution progressive multifocale du CNS ou l'évolution récidivante et rémittente à site unique.

22. Utilisation suivant la revendication 1, dans laquelle le médicament anti-inflammatoire ou immunomodulateur est sélectionné parmi le groupe comprenant un dérivé d'interféron, un dérivé de prostane, un glucocorticoïde, un immunosuppresseur, un inhibiteur de lipoxygénase, un antagoniste de leucotriène, une substance anti-inflammatoire, un dérivé peptidique médicamenteux ou un calcipotriol ou un analogue de celui-ci.

23. Utilisation suivant la revendication 1 ou 22, dans laquelle le médicament est un dérivé d'interféron.
